# EUROPEAN PATENT SPECIFICATION

(11) **EP 1 454 891 B1**
(45) Date of publication and mention of the grant of the patent: **28.12.2005**
(21) Application number: 03251417.6
(22) Date of filing: 07.03.2003
(51) Int. Cl.: C07C 41/24, C07C 41/01, C07C 51/265, C07C 65/24, C07C 43/275

(54) **Process for preparation of 4-(4-phenoxyphenoxy) benzoic acid by oxidation of 1-(4-methylphenoxy)-4-phenoxybenzene**
Verfahren zur Herstellung von 4-(4-Phenoxy-phenoxy)-Benzoesäure durch Oxidation von 1-(4-Methylphenoxy)-4-Phenoxybenzol
Procédé de préparation de l'acide 4-(4-phénoxyphénoxy) benzoique par oxidation de 1-(4-méthylphénoxy)-4-phénoxybenzène

(43) Date of publication of application: 08.09.2004
(73) Proprietor: Gharda Chemicals Limited, Bandra (W), Mumbai - 400 050 (IN)
(72) Inventor: Gharda, Keki Hormushi, Gharda Chemicals Limited, Dombivli,421203, Dist. Thane, Maharastra (IN); Joseph, Pulinattu Cherian, Gharda Chemicals Ltd, Dombivli,421203, Dist. Thane, Maharastra (IN); Joshi, Prakash Achyut, Gharda Chemicals Limited, Dombivli,421203, Dist. Thane, Maharastra (IN); Kalyana Sundaram, Pallavur Sankaranarayanan, Dombivli,421203, Dist. Thane, Maharastra (IN)
(74) Representative: Wise, Stephen James

(56) References cited:
- DATABASE WPI Section Ch, Week 198823 Derwent Publications Ltd., London, GB; Class E14, AN 1988-157204 XP002247827 & JP 63 096151 A (ASAHI CHEM IND CO LTD), 27 April 1988 (1988-04-27)

## Description

This invention relates to a process for producing 4-(4-phenoxyphenoxy)benzoic acid by a novel oxidation process hitherto not reported.
4-(4-phenoxyphenoxy)benzoic acid is a useful monomer for preparing polymers, particularly poly ether ether ketone (PEEK), a speciality thermoplastic polymer used in high temperature, resistant to; chemical, wear and abrasion applications. This patent covers the preparation of the intermediate used for oxidation, viz 1-(4-methyl phenoxy),4-phenoxy benzene by the nucleophilic condensation process starting from commonly available raw materials like phenol, para cresol and para dihalobenzene in a one pot process, preferably, in two separate steps.

The above mentioned polymer PEEK is currently manufactured by Victrex Plc, the only manufacturer in the world known to be, by the nucleophilic process involving condensation of 4,4'-difluorobenzophenone and hydroquinone at high temperatures of above 300°C which requires the use of costly raw materials like 4,4'-difluorobenzophenone and high temperature operation. Alternate process by the electrophilic route starting from the monomer 4-(4-phenoxyphenoxy)benzoic acid or it's derivatives are reported in literature, which occurs at lower temperatures, i.e., below 100°C. We have chosen the low temperature elecrophilic route for which the monomer 4-(4-phenoxyphenoxy) benzoic acid is critical and is the subject matter of this invention by the hitherto unreported air oxidation process. Our two patent applications 333/BOM/99 dated 4.5.1999 with Indian Patent Office and 0016527.4 dated 6.7.2000 with UK Patent Office on the polymerization process using the said monomer is pending allotment.

There had been known methods of making 4-(4-phenoxyphenoxy)benzoic acid, for example, Japanese Patent 63096151 which dealt with making of the said compound by a method in which di lodo diphenyl ether is reacted with carbon monoxide in a catalyst system and the resultant lodo phenoxy benzoic acid derivative is reacted with sodium phenate in a Cul catalyzed system. This method suffers from a number of drawbacks like handling of poisonous carbon monoxide gas at high temperatures, use of costly lodo compounds etc. In short, this may be a method for making small quantities in the laboratory for further testing of polymer synthesis but cannot be employed for a manufacturing process due to above and economic reasons.

Our synthetic expertise and experience in liquid phase air oxidations led us to believe that the best way to make this compound is by the cost effective air oxidation route starting from 1-(4-methylphenoxy),4-phenoxybenzene. Thus, a detailed synthetic project was undertaken for preparation of this key monomer for the polymer PEEK, starting with the synthesis of the intermediate, which was hitherto unreported.

It has been known in literature that alkali phenolates can be condensed with aromatic halides using cuprous halides to result in aromatic ethers. It has also been known that aromatic alkyl compounds can be oxidized using the well-known CO++/Br- catalyst system to yield carboxylic acid derivatives. Our invention is, particularly, preparation of 4-(4-phenoxyphenoxy)benzoic acid, using the known reactions, on a new synthetic methodology hitherto not reported.

The present invention relates to a method of preparation of 4-(4-phenoxyphenoxy)benzoic acid by air/oxygen by oxidation of the compound 1-(4-methylphenoxy),4-phenoxybenzene and its preparation from basic raw materials.
The chemical reactions involved in the present invention are as follows :

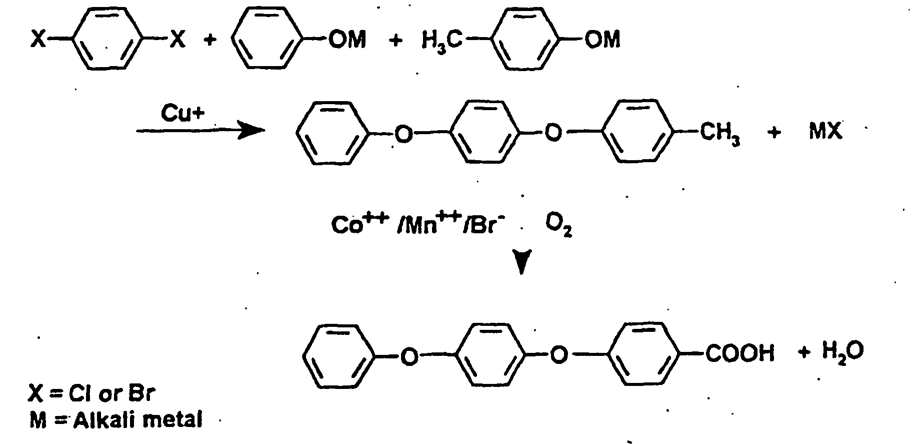

According to the invention, para dihalo benzene is reacted with an alkali phenolate using cuprous halide catalyst. The halogens of the para dihalo benzene may be F, Cl, Br or I but preferably Cl or Br due to reactivity and economic considerations. The alkali used for phenolate formation may be Li. Na, K, Ca, Mg etc. The preferred alkali is Na or K, particularly preferred alkali being Na from the reactivity and economic considerations. The para dihalobenzene is preferred to be condensed with alkali phenolate first, due to economic considerations; however, we found that in the case of Para dichloro benzene, the first reaction, using sodium para cresolate was preferred due to higher overall yields and in the case of Para dibromo benzene, sodium phenate could be used for the first condensation. The catalyst used can be any form of copper or its salts particularly preferred salts being cuprous chloride or cuprous bromide. Use of an organic N compound, though not essential, helps to hasten catalysis rate and helps the mass to be more stirrable due to solubility of the salts. The organic N compound may be Dimethyl formamide (DMF), Dimethylacetamide (DMAC), N-Methyl Pyrollidone (NMP), Pyridine, Quinoline etc and is not limited to the ones mentioned in examples. The quantity of the N compound is limited to the minimum needed, for economic reasons of recovery reuse etc.
The temperature of the reaction can be in the range of 120°C-250°C, the preferred range being 165-175°C. Anhydrous condition is most preferred for the reaction, though it can also be done in aq. conditions at loss of efficiency.

The halo diphenyl ether thus produced is condensed with alkali phenolate or alkali paracresolate as the case may be under more or less the same conditions as the earlier step was carried out. The reaction can be carried out in the temperature range of 170-275°C, the preferred temperature being 180-200°C in the case of chloro deriv. All other conditions with regard to alkali metal, copper catalyst, organic N compound, etc is applicable for this step as in the earlier step. The compound is fractionally distilled to yield high purity required for the next step, which is oxidation. The oxidation can be effected by any source of any oxygen such as gaseous oxygen, an oxidant that yields oxygen, or an oxygen-containing gas, such as air, preferably under pressure.

The above two reaction steps can also be done, in a single pot where the product from first step is not isolated but taken for the second step. However, as a good manufacturing practice, purity and yield of product, the two steps are carried out separately and this is the preferred way.
Alternately, the key intermediate, 1-(4-methylphenoxy),4-phenoxybenzene may be made by condensing hydroquinone salt sequentially with bromobenzene and 4-bromotoluene or variations thereof, viz. use of para chloro phenol or para bromophenol in place of hydroquinone etc. In short, the ether linkage is formed by reaction of a halo benzene derivative with a phenolic salt and the variations thereof should not be interpreted as a new invention for preparation of the compound, 1-(4-methylphenoxy),4-phenoxybenzene.

The oxidation of the intermediate 1-(4-methylphenoxy),4-phenoxybenzene to 4-(4-phenoxyphenoxy) benzoic acid is carried out using air or oxygen under pressure in propionic or acetic acid medium containing transition metal and bromide catalyst system. The medium for oxidation can be any organic compound which do not undergo oxidation or interfere with the oxidation process; the preferred compounds are benzoic acid, chlorobenzene, chlorobenzoic acid, aliphatic acids like acetic, propionic, butyric etc, and the most preferred in this case being acetic acid or propionic acid.
The oxidation can be done using oxygen or air under pressure, the function of pressure being to increase the partial pressure of oxygen and at higher pressures, the oxidation rate increases. The preferred range is 15-50 kg/cm2 air pressure, preferably 20-25 kg/cm2 from practical considerations. The oxygen input and rate of oxidation is controlled mainly from the heat removal aspect. Faster oxidation with adequate heat removal facility leads to better yield and higher purity.

The catalyst system Cobalt salt with or without Manganese salt in combination with bromide is well known in literature for oxidations of many compounds.

However, oxidation of 1-(4-methylphenoxy), 4-phenoxybenzene by the method described here is our invention which has significant commercial importance. The oxidation can be carried out at a wide range of temperatures like 100 - 250°C, the preferred range being 120-150°C.

The following examples are intended to aid in the understanding of the invention and variations may be made by one skilled in the art without departing from the spirit and scope of the invention.

### EXAMPLE-1

108 g para cresol is charged to 600 g para dichlorobenzene. 80 g 50% caustic soda lys is added to it and the mass is kept stirred and distilled to remove the water at liquid temperature 140-170°C. 50ml of N-Methyl pyrollidone and 5 g CuCl are added to the mass and continued stirring at 170°C for 8 has till the reaction is complete. The mass is cooled and worked up by filtration of NaCl at 50°C, and the filtrats is taken for distillation of excess Para dichlorobenzene and the product is fractionally distilled at 175°C at 10 mm Hg pr., resulting in 187 g (87% yield), 4-chlorophenyl-4'-methylphenyl ether, m.p. 53°C.

### EXAMPLE-2

To a stirred reaction flask having water distin and reflux arrangement are added 200 ml toluene, 200 g phenol, 80 g 50% caustic soda lye and the mass is heated and water is removed by azeotropic distillation. To this is added 220 g 4-chlorophenyl-4'-methylphenyl ether prepared in Example-1 50 ml N-methylpyrollidone and 5 g CuCl. The change is heated to 200°C liquid temperature under stirring with distillation of toluene and maintained at 200°C for 8 hrs till reaction is complete. The mass is cooled to 70°C, filtered off NaCl and the filtrate is taken for fractional distilation at 2 mmHg pr at vapour temperature 190-192°C, resulting in 235 g product 1-(4-methyl phenoxy), 4-phenoxy benzene (85% yield) having m.p. 52-53°C.

### EXAMPLE-3

To 200 ml toluene, 94g phenol and 80 g 50% caustic soda lye are added and water is azeotropically distilled out. 50 ml DMF and 475 g paradibromo benzene (PDBB) and 5 g cuprous bromide are added to it. The mass is maintained at 160-165°C for 5 hours by distilling toluene. The mass is filtered at 80°C, washed with toluene and the filtrate fractionally distilled under vacuum to give 229 g 4-bromodiphenyl ether, 99% purity, b.p. - 125°C/2 mm.

### EXAMPLE-4

To 260 ml toluene, 108 g para cresol and 80 g 50% caustic lye are added and the water is azeotropically distilled out. To the mass 50 ml DMF, 249 g 4-bromodiphenyl ether and 5 g CuBr are added. The mass is maintained at 150-165°C for 6 hrs, cooled to 100°C and filtered. The filtrate on fractional distillation gave 235 g (85% yield) of 1-(4-methylphenoxy),4-phenoxybenzene, b.p. 200-201°C/4 mm Hg pr., m.p. - 53-54°C.

### EXAMPLE-5

The oxidation assembly consists of a glass vessel, 6" dia and 8" height, with a condenser, gas inlet tube, thermowell and motor driven agitator capable of operating at 1.5 kg/cm2 pressure. Into this reactor is charged 850 ml propionic acid, 12.5 g cobalt acetate, 12.5 g manganese acetate, 10 g NH4Br and 276 g 1-(4-methylphenoxy),4-phenoxybenzene. The mixture is heated to 130°C and oxygen is passed into the reactor at 1.5 kg/cm2 pr at the rate of 0.25 moles per hr with the exit valve opened to control the pressure and flow rate. It is continued for a total of 12 hrs when the oxidation is complete and no intake of oxygen is there. The pressure is released and the contents are slowly cooled to 40°C. The product precipitates out. It is filtered and washed with propionic acid. The wet cake is recrystallized from 1000 ml propionic acid by heating to 100°C to dissolve, cooling to 45°C and filtration. The cake is washed with water and dried to yield 230 g 4-(4-phenoxyphenoxy)benzoic acid in 75% yield and purity of 99.6% by HPLC, m.p. : 184°C, is suitable for use in polymerization.

### EXAMPLE-6

The oxidation assembly consists of a 2 litre capacity autoclave, made of Titanium metal operating pressure 50 kg/cm2, gas inlet tube, condenser, thermowell, highspeed agitator system etc. In the autoclave, a charge consisting of 1000 ml acetic acid, 12.5 g cobalt acetate, 12.5 g manganese acetate, 10 g NH4Br and 276 g 1-(4-methylphenoxy),4-phenoxybenzene is charged. The charge is heated to 130-140°C and air is passed at the rate of 1.5 m/hr maintaining a pressure of 25 kg/cm2 with continuous venting of the exit gases from the top of condenser. The oxidation is continued for 12 hrs till there is no more intake of oxygen. The pressure is released and mass is cooled to 40°C. It is filtered and washed with acetic acid. The wet cake is re-crystallized from 1000 ml acetic acid by dissolving at 100-105°C and slowly cooling to 40°C. This is filtered, washed with acetic acid, water and dried at 100°C to yield 224 g 4-(4-phenoxyphenoxy)benzoic acid in 73% yield and 99.5% purity by HPLC, m.p. 184°C, is suitable for polymerization.

### EXAMPLE-7

An oxidation batch is carried out as per procedure in example-6 in which oxidation is carried out at a faster rate. Thus, keeping all other conditions same the air rate was changed to 4.5m/hr. The oxidation was completed in 4 hours and the workup resulted in 233 g 4-(4-phenoxyphenoxy)benzoic acid, in 76% yield and 99.7% purity by HPLC. The filtrate workup and isolation of a second crop resulted in 33 g of the product which is 1% of the yield having purity of 99.6% by HPLC.

## Claims

1. A process for the production of monomer C for thermoplastic aromatic poly ether ketone **characterized in that** said process comprises following steps :
a) Preparation of aromatic mono ether of the following formula: where X = Cl or Br and R = H or Methyl. 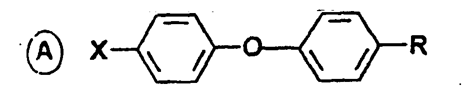
b) Preparation of the intermediate 1-(4-methylphenoxy),4-phenoxybenzene 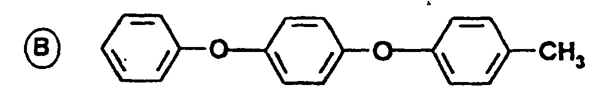 from the above intermediate A.
c) Oxidation of the above intermediate B to give the corresponding carboxylic acid,

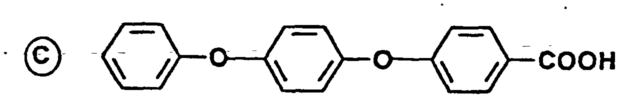

2. A process according to claim 1 (a) in which para dichloro benzene is reacted with alkali para cresolate to give 4-chlorophenyl-4'-methylphenyl ether, using CuCl catalyst.

3. A process according to claim 1 (a) in which para dibromobenzene is reacted with alkali phenolate to give 4-bromophenoxy benzene, using CuCl as catalyst.

4. A process according to claim 2 where the para dichloro benzene is reacted with alkali phenolate.

5. A process according to claim 3, where the para dibromo benzene is reacted with alkali para cresolate.

6. A process according to claim 1 (b), where the intermediate (B) is made from intermediate (A) by reaction with alkali paracresolate or alkali phenolate as the case may be using CuCl catalyst.

7. A process according to claims 1 - 6, where during reaction, along with the catalyst CuCl, NMP, DMF or other aryl/alkyl amide is added as catalyst aid.

8. A process according to claim 1(c), where intermediate (B) is oxidized in acetic, propionic or other aryl alkyl acids using catalyst cobalt salt alone or in combination with manganese salt and a bromide source.

9. A process according to claim 8, where oxidation is done using oxygen or air under pressure and purification of the product resulting in 4-(4-phenoxy phenoxy) benzoic acid of purity acceptable for use in polymerization.

## Patentansprüche

1. Verfahren zur Herstellung von Monomer C für thermoplastisches aromatisches Polyetherketon, **dadurch gekennzeichnet, dass** das Verfahren die folgenden Schritte umfasst:
a) Herstellung von aromatischem Monoether der folgenden Formel: wo X = Cl oder Br und R = H oder Methyl ist 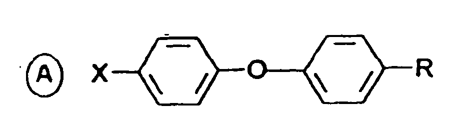
b) Herstellung der Zwischenverbindung 1-(4-Methylphenoxy),4-phenoxybenzol 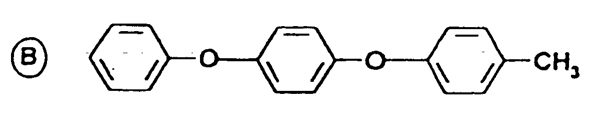 aus der obigen Zwischenverbindung A,
c) Oxidation der obigen Zwischenverbindung B, so dass die entsprechende Carbonsäure erhalten wird

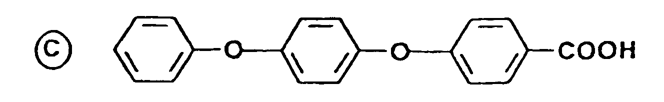

2. Verfahren nach Anspruch 1(a), in dem Paradichlorbenzol mit Alkaliparacresolat unter Verwendung von CuCl als Katalysator umgesetzt wird, so dass 4-Chlorphenyl-4'-methylphenylether erhalten wird.

3. Verfahren nach Anspruch 1(a), in dem Paradibrombenzol mit Alkaliphenolat unter Verwendung von CuCl als Katalysator umgesetzt wird, so dass 4-Bromphenoxybenzol erhalten wird.

4. Verfahren nach Anspruch 2, wo Paradichlorbenzol mit Alkaliphenolat umgesetzt wird.

5. Verfahren nach Anspruch 3, wo Paradibrombenzol mit Alkaliparacresolat umgesetzt wird.

6. Verfahren nach Anspruch 1(b), wo die Zwischenverbindung (B) aus Zwischenverbindung (A) durch Umsetzung je nach dem mit Alkaliparacresolat oder Alkaliphenolat unter Verwendung von CuCl als Katalysator gebildet wird.

7. Verfahren nach den Ansprüchen 1-6, wo bei der Umsetzung zusammen mit dem Katalysator CuCl, NMP, DMF oder anderes Aryl/Alkylamid als Hilfskatalysator zugesetzt wird.

8. Verfahren nach Anspruch 1(c), wo Zwischenverbindung (B) in Essig-, Propion- oder anderer Aryl/Alkylsäure unter Verwendung von Katalysator Cobaltsalz allein oder in Kombination mit Mangansalz und einer Bromquelle oxidiert wird.

9. Verfahren nach Anspruch 8, wo Oxidation unter Verwendung von Sauerstoff oder Luft unter Druck vorgenommen wird und Reinigung des Produkts zu 4-(4-Phenoxyphenoxy)benzoesäure führt, deren Reinheit zur Verwendung bei der Polymerisation geeignet ist.

## Revendications

1. Procédé de production du monomèrec pour polyéthercétone aromatique thermoplastique **caractérisé en ce que** ledit procédé comprend les étapes suivantes :
a) Préparation de mono-éther aromatique de formule suivante : dans laquelle X = Cl ou Br et R = H ou méthyle 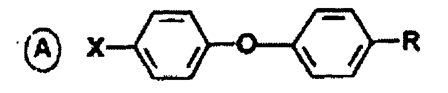
b) Préparation de l'intermédiaire 1-(4-méthylphénoxy), 4-phénoxybenzène 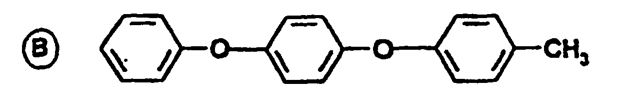 à partir de l'intermédiaire A ci-dessus.
c) Oxydation de l'intermédiaire B ci-dessus pour donner l'acide carboxylique correspondant :

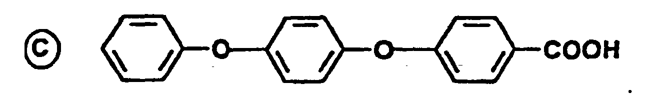

2. Procédé selon la revendication 1(a) dans lequel on fait réagir le paradichlorobenzène avec un paracrésolate alcalin pour donner le 4-chlorophényl-4'-méthylphényléther, en utilisant un catalyseur CuCl.

3. Procédé selon la revendication 1(a) dans lequel on fait réagir le paradibromobenzène avec un phénolate alcalin pour donner le 4-bromo-phenoxy benzène, en utilisant un catalyseur CuCl.

4. Procédé selon la revendication 2 dans lequel on fait réagir le paradichlorobenzène avec un phénolate alcalin.

5. Procédé selon la revendication 3, dans lequel on fait réagir le paradibromo-benzène avec un paracrésolate alcalin.

6. Procédé selon la revendication 1(b), dans lequel on fabrique l'intermédiaire (B) à partir de l'intermédiaire (A) par réaction avec un paracrésolate alcalin ou un phénolate alcalin en utilisant éventuellement suivant les cas un catalyseur CuCl.

7. Procédé selon les revendications 1 à 6, dans lequel pendant la réaction, en même temps que le catalyseur CuCl, NMP, DMF ou un autre aryl/alkyl-amide est ajouté comme co-catalyseur.

8. Procédé selon la revendication 1(c), dans lequel on oxyde l'intermédiaire (B) dans les acides acétique, propionique ou d'autres arylalkyl en utilisant comme catalyseur un sel de cobalt seul ou en combinaison avec un sel de manganèse et une source de bromure.

9. Procédé selon la revendication 8, dans lequel on effectue l'oxydation en utilisant de l'oxygène ou de l'air sous pression et la purification du produit pour obtenir l'acide 4-(4-phénoxyphénoxy)benzoïque de pureté acceptable pour une utilisation en polymérisation.
